(19) Europäisches Patentamt
European Patent Office
Office européen des brevets

(11) Numéro de publication: **0 039 646**
**A1**

# (12) DEMANDE DE BREVET EUROPEEN

(21) Numéro de dépôt: **81400710.0**

(22) Date de dépôt: **06.05.81**

(51) Int. Cl.³: **C 07 D 405/06**, A 61 K 31/335

(30) Priorité: **07.05.80 FR 8010223**

(43) Date de publication de la demande: **11.11.81**
**Bulletin 81/45**

(84) Etats contractants désignés: **AT BE CH DE FR GB IT LI LU NL SE**

(71) Demandeur: **Centre Européen de Recherches Mauvernay (CERM), Route de Marsat, F-63201 Riom (FR)**

(72) Inventeur: **Coudert, Gérard, 232, Avenue du Général Leclerc, F-54000 Nancy (FR)**
Inventeur: **Guillaumet, Gérald, 22, rue Victor-Basch, F-54500 Vandoeuvre (FR)**
Inventeur: **Lalloz, Lucien, 25, rue du Chanoine Jacob, F-54000 Nancy (FR)**
Inventeur: **Loppinet, Vincent, 45, Avenue Foch, F-54000 Nancy (FR)**

(74) Mandataire: **Therond, Gérard Raymond, C.E.R.M Route de Marsat, F-63201 Riom (FR)**

## (54) Nouvelles benzo (1,4) dioxines, leurs méthodes de préparation et leur application en thérapeutique.

(57) L'invention concerne des benzo (1,4) dioxines de formule:

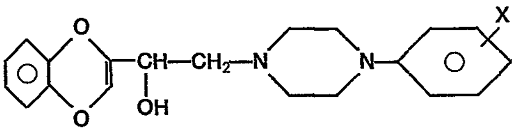

et leurs sels pharmaceutiquement acceptables dans laquelle X représente l'hydrogène, un radical alkyle ou alcoxy inférieur ou le radical trifluorométhyle.
Application comme antihypertenseur.

EP 0 039 646 A1

NOUVELLES BENZO (1,4) DIOXINES, LEURS METHODES DE PREPARATION ET LEUR APPLICATION EN THERAPEUTIQUE

La présente invention concerne de nouvelles benzo (1,4) dioxines substituées en 2 par une chaîne amino-alcool, répondant à la formule générale suivante:

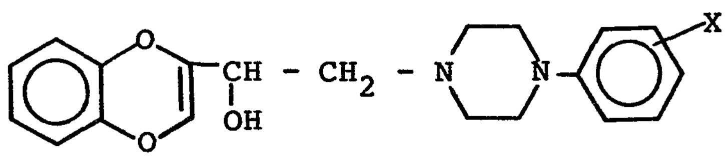

dans laquelle X représente l'hydrogène, un radical alkyle ou alcoxy inférieur, ou le radical trifluorométhyle. Selon une réalisation préférée X représente le radical méthoxy en position ortho.

L'invention concerne aussi l'application de ces composés, et de leurs sels pharmaceutiquement acceptables en thérapeutique, notamment en raison de leurs intéressantes propriétés antihypertensives.

Un autre objet de l'invention consiste dans le procédé particulier permettant de synthétiser ces composés par exemple à partir d'une 2-haloformyl benzo (1,4) dioxine. Ce procédé en trois étapes peut être schématisé comme suit :

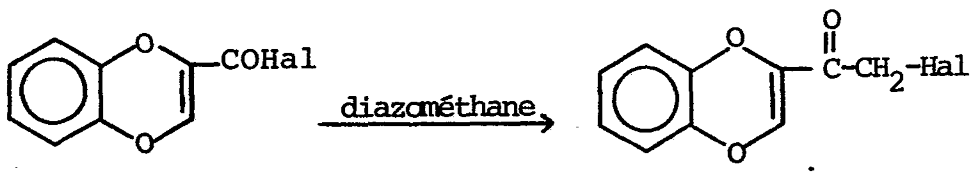


(Hal représente un halogène de préférence le chlore)

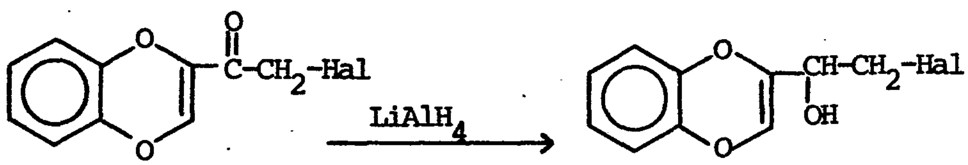


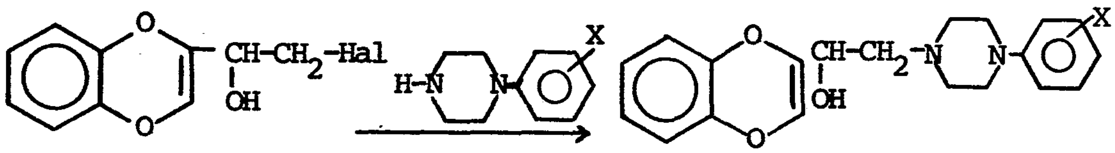

La 2-haloformyl benzo (1,4) dioxine utilisée comme matière première peut elle-même être préparée selon une méthode connue consistant par exemple à traiter le 2-carbéthoxy benzo (1,4) dioxanne par le N-bromosuccinimide, puis le 2,3 dibromo 2-carbéthoxy benzo (1,4) dioxanne obtenu par l'iodure de sodium, et à transformer par les moyens habituels (hydrolyse, acidification par HCl et traitement par $SOCl_2$) la 2-carbéthoxy benzo (1,4) dioxine en 2-haloformyl benzo (1,4) dioxine.

Le procédé selon l'invention comprend donc plus précisément :

a) l'addition progressive de 2-chloroformyl benzo (1,4) dioxine à une solution éthérée de diazométhane, avec maintien du mélange réactionnel entre - 20 et - 10°C, et ensuite le maintien à la température ambiante, avec agitation, pendant environ 14 heures et l'addition d'acide chlorhydrique en solution saturée dans l'éther, jusqu'à disparition de la diazocétone formée ;

b) la dissolution dans l'éther anhydre et la réduction de la 2-chloroacétyl benzo (1,4) dioxine précédemment obtenue ; et

c) la réaction du chloroalcool précédemment obtenu avec une 4-phenyl piperazine substituée par X tel que précédemment défini, en milieu solvant tel que l'hexamétapol ainsi que, si on le désire, la conversion de la base ainsi obtenue en un sel pharmaceutiquement acceptable.

Pour obtenir les sels pharmaceutiquement acceptables de ces composés, les bases obtenues sont traitées avec des acides pharmaceutiquement acceptables tels que l'acide chlorhydrique, l'acide maléique, l'acide tartrique, l'acide fumarique, l'acide citrique etc... Le chlorhydrate par exemple est obtenu en traitant la base par une solution d'éther saturée en acide chlorhydrique.

L'activité pharmacologique des composés de l'invention a été révélée par recherche d'une interférence avec les récepteurs adrénergiques $\beta_1$, $\beta_2$ et $\alpha$ in vitro, et confirmée in vivo par les effets antihypertenseurs observés après administration desdits composés chez le rat spontanément hypertendu anesthésié.

Les essais effectués in vitro correspondent aux protocoles résumés ci-après :

- Récepteurs adrénergiques $\beta_1$ : on recherche l'antagonisme de l'effet inotrope positif de l'isoprénaline sur les récepteurs $\beta_1$ de l'oreillette gauche de cobaye stimulée électriquement.

- Récepteurs adrénergiques $\beta_2$ : on recherche l'antagonisme des effets bronchodilatateurs de l'isoprénaline sur les récepteurs $\beta_2$ du muscle trachéal de cobaye.

- Récepteurs adrénergiques $\alpha$ : on étudie l'interférence des composés de l'invention sur la contraction du vas déferens isolé du rat, provoquée par des doses cumulatives de noradrénaline.

Les résultats exprimés par les paramètres usuels de pharmacologie moléculaire, selon les critères utilisés par Van Rossum [Arch. Int. Pharmacodyn., 143, 299-330, 1963] sont les suivants pour le composé de l'exemple 2 :

Récepteurs $\beta_1$ : $pA_2 = 6,04$ , $pD'_2 = 4,84$.

Récepteurs $\beta_2$ : inactif.

Récepteur $\alpha$ : à la dose de $10^{-4}$ M/l, potentialisation de 180 % des effets de la noradrénaline.

Le composé de l'exemple 1 a une action très faiblement compétitive à $10^{-4}$ M/l sur les récepteurs $\beta_1$, et il est inactif sur les récepteurs $\beta_2$ et $\alpha$ .

Les essais in vivo ont été effectués chez des rats mâles (S.H.R. - I.F.F.A.) conformément au protocole résumé ci-après :

Après anesthésie au pentobarbital, on a disposé un cathéter au niveau de l'artère carotide pour enregistrer la pression artérielle, et un cathéter au niveau d'une veine jugulaire pour permettre les injections des composés de l'invention mis en solution dans 0,5 ml de PEG 200 (polyéthylène glycol). La durée de l'injection était de 1 minute.

Avec le composé n° 2, à la dose de 2,5 $mg.kg^{-1}$, on a observé un effet antihypertenseur durable pendant une durée supérieure à 45 minutes.

Avec le composé n° 1, cet effet antihypertenseur a été observé pendant 20 minutes pour une dose administrée de 20 $mg.kg^{-1}$.

Ces résultats montrent en particulier le grand intérêt du composé n° 2 qui présente un antagonisme très sélectif du type dualiste vis-à-vis des récepteurs $\beta$ adrénergiques et fait preuve d'une remarquable activité in vivo chez le rat spontanément hypertendu anesthésié.
Il a d'autre part été possible d'évaluer à 120 $mg.kg^{-1}$ la toxicité aiguë du composé n° 2 par voie orale chez la souris.

Ces produits peuvent donc être appliqués en thérapeutique humaine en tant qu'antihypertenseurs. Associés aux excipients pharmaceutiques habituels, ils peuvent être administrés par voie enthérale ou parenthérale à des doses journalières comprises entre 0,1 mg et 50 mg par kg de poids corporel. La dose préférée chez l'homme est comprise entre 50 et 500 mg.

Les composés préférés de l'invention sont les composés de formule I dans lesquels X est un substituant en position ortho et représente un groupe alcoxy ayant de 1 à 4 atomes de carbone, tels que les radicaux méthoxy, éthoxy, n-propoxy, isopropoxy ou n-butoxy. La signification préférée du substituant X est le radical méthoxy.

Le procédé est illustré plus en détail par référence aux exemples ci-après.

En préliminaire à ces exemples, on rappelle tout d'abord un moyen permettant de synthétiser le 2-chloroformyl benzo (1,4) dioxine utilisé comme matière première pour la synthèse de tous les composés de l'invention.

Un réacteur contenant 300 ml de tétrachlorure de carbone anhydre, 62,4 g (0,3 M) de 2-carbéthoxy benzo (1,4) dioxanne, 120 g (0,66 M) de N-bromosuccinimide et 2,0 g de peroxyde de benzoyle a été porté à reflux pendant 6 à 7 heures.

Lorsque la réaction a été terminée, on a laissé le milieu réactionnel revenir à température ambiante, filtré le précipité de succinimide, lavé avec du tétrachlorure de carbone, puis chassé les solvants.

On a ainsi obtenu 100 g de 2,3-dibromo 2-carbéthoxy benzo (1,4) dioxanne ayant un point de fusion F = 88°C.

Le produit brut ainsi obtenu a été dissous dans 600 ml d'acétone anhydre, puis traité par 180 g d'iodure de sodium anhydre, tandis qu'on maintenait le milieu réactionnel sous agitation, à température ambiante, pendant environ 1 heure et demie.

Après évaporation de l'acétone, la pâte verdâtre obtenue a été reprise par 400 ml d'eau, 300 ml d'éther et 600 ml d'une solution normale d'hyposulfite de sodium.

Après séparation, purification et séchage de la phase organique, élimination de l'éther par évaporation et recristallisation dans le pentane, on a obtenu 58 g de 2-carbéthoxy benzo (1,4) dioxine ayant un point de fusion F = 39 - 40°C.

L'ester a ensuite été traité à 95°C par une solution de soude à 8 %, puis par l'acide chlorhydrique concentré. Après filtration, lavage à l'eau, puis à l'éther de pétrole, séchage sous vide et recristallisation dans le benzène, on a obtenu 45 g d'acide 2-carboxy benzo (1,4) dioxine ayant pour point de fusion 176°C.

Pour obtenir le chlorure d'acide utilisé comme matière première dans la présente synthèse, on a traité l'acide précédent, mis en solution dans 300 ml de benzène anhydre, par 90 g de chlorure de thionyle en maintenant le reflux pendant environ 14 heures. Après évaporation du solvant, on a obtenu 48 g de 2-chloroformyl benzo (1,4) dioxine ayant un point de fusion F = 106 - 107°C.

EXEMPLE 1 : 2-méthanolα-[4-(3-trifluoromethyl phenyl) piperazinyl-1] methyl benzo (1,4) dioxine

Dans un réacteur contenant une solution de diazométhane précédemment préparée à partir de 28 g de N-nitrosométhylurée, 38 g de potasse, 64 ml d'eau et 280 ml d'éther et maintenue entre - 20 et - 10°C, on a ajouté goutte à goutte une solution de 16,0 g (0,081 M) de 2-chloroformyl benzo (1,4) dioxine dans 350 ml d'éther. L'addition étant terminée, on a laissé l'agitation se poursuivre 30 minutes à froid, puis 14 heures à température ambiante. La diazocétone formée a ensuite été déplacée par addition rapide d'une solution éthérée saturée d'acide chlorhydrique. Lorsque la réaction a été terminée, on a lavé la phase éthérée avec une solution saturée de bicarbonate de sodium, puis à l'eau. Après séchage sur sulfate de sodium et évaporation de l'éther, le produit a été repris par 400 ml de benzène-éther de pétrole (1/4) et la solution obtenûe filtrée à chaud. Après évaporation des solvants et lavage des cristaux avec 75 ml d'éther-éther de pétrole (1/2), on a obtenu 13,5 g de 2-chloroacétyl benzo (1,4) dioxine ayant un point de fusion F = 91 - 92°C.

Dans une deuxième étape, on a dissous 10,50 g (0,050 M) de la chlorocétone précédemment obtenue dans 400 ml d'éther anhydre, puis, la solution étant maintenue sous agitation à une température inférieure à

20°C, on a ajouté l'hydrure de lithium et d'aluminium jusqu'à ce que la réduction soit achevée (fin de réaction contrôlée par chromatographie sur couche mince). Après hydrolyse avec un minimum d'eau, séchage sur sulfate de magnésium et filtration, on a chassé l'éther sous pression réduite et obtenu 10,5 g de 2-méthanol α-chlorométhyl benzo (1,4) dioxine sous forme d'une huile légèrement jaune.

Dans la troisième étape, on a dissous 3,80 g (18 mM) du chloro-alcool précédemment obtenu et 10 g (45 mM) de 4-(3-trifluoromethyl phenyl) piperazine dans 50 ml d'hexamétapol, et maintenu la solution sous agitation à 85°C pendant 40 heures.

On a ainsi obtenu 4,20 g de composé du titre qui après recristallisation dans le benzène avait pour point de fusion 140°C, et pour analyse élémentaire :

| | C % | H % | N % |
|---|---|---|---|
| Trouvé | 62,62 | 5,18 | 6,85 |
| Théorique | 62,13 | 5,21 | 6,90 |

EXEMPLE 2 : 2-méthanolα-[4-(2-methoxy phenyl) piperazinyl-1] methyl benzo (1,4) dioxine

On a d'abord préparé comme indiqué dans l'exemple 1 la 2-méthanolα-chlorométhyl benzo (1,4) dioxine. Dans un réacteur, on a mis en solution 5,25 g (25 mM) du chloroalcool et 12,2 g (62,5 mM) de 4-(2-methoxy phenyl) piperazine dans 15 ml de tétrahydrofuranne et 30 ml d'hexamétapol, et on a maintenu la solution sous agitation à 85°C pendant 30 heures.

On a ainsi obtenu 5 g de composé du titre qui après recristallisation dans le benzène avait pour point de fusion 102°C, et pour analyse élémentaire :

| | C % | H % | N % |
|---|---|---|---|
| Trouvé | 68,01 | 6,63 | 7,58 |
| Théorique | 68,54 | 6,57 | 7,61 |

De la même façon, on a préparé la 2-méthanol α-[4-phenyl piperazinyl-1] methyl benzo (1,4) dioxine.

REVENDICATIONS

1. Benzo (1,4) dioxines substituées répondant à la formule :

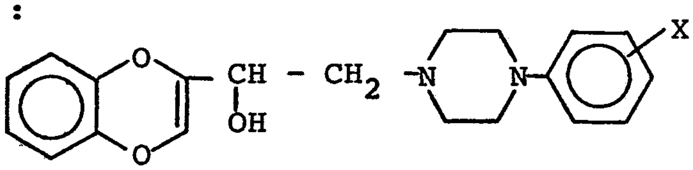

dans laquelle X représente l'hydrogène, un radical alkyle ou alcoxy inférieur ou le radical trifluorométhyle,et leurs sels pharmaceutiquement acceptables.

2. Composé selon la revendication 1 caractérisé en ce que X représente le radical méthoxy en position ortho.

3. Procédé pour la préparation d'un composé selon la revendication 1 caractérisé en ce que le composé

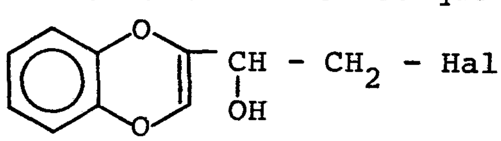

dans lequel Hal représente un halogène, de préférence le chlore, est mis à réagir avec un composé de formule :

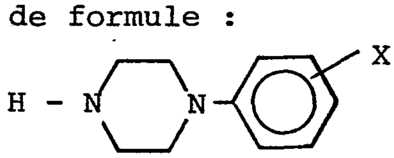

ou l'un de ses sels d'addition, dans lequel X a la signification précédemment indiquée, puis est tranformé en un sel d'addition pharmaceutiquement acceptable.

4. Application des composés selon les revendications 1 ou 2 en tant que produits antihypertenseurs.

5. Composition pharmaceutique contenant à titre de principe actif un composé selon les revendications 1 ou 2, associé avec un ou plusieurs excipients pharmaceutiques habituels.

0039646

Numéro de la demande

Office européen des brevets

# RAPPORT DE RECHERCHE EUROPEENNE

EP 81 40 0710

| DOCUMENTS CONSIDERES COMME PERTINENTS | | | CLASSEMENT DE LA DEMANDE (Int. Cl.³) |
|---|---|---|---|
| Catégorie | Citation du document avec indication, en cas de besoin, des parties pertinentes | Revendication concernée | |
| | FR - M - 3 579 (ICI)<br>* En entier *<br>-- | 1,4,5 | C 07 D 405/06<br>A 61 K 31/335 |
| | US - A - 3 928 358 (BOEHRINGER)<br>* Abrégé ; colonnes 1-4,9-12 *<br>--- | 1-5 | |

DOMAINES TECHNIQUES RECHERCHES (Int. Cl.³)

C 07 D 319/20
A 61 K 31/335
C 07 D 405/06

CATEGORIE DES DOCUMENTS CITES

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons

&: membre de la même famille, document correspondant

Le présent rapport de recherche a été établi pour toutes les revendications

| Lieu de la recherche | Date d'achèvement de la recherche | Examinateur |
|---|---|---|
| La Haye | 21-07-1981 | FRANCOIS |